# EUROPEAN PATENT APPLICATION

(11) **EP 2 062 578 A1**
(43) Date of publication of application: **27.05.2009**
(21) Application number: 07021911.8
(22) Date of filing: 12.11.2007
(51) Int. Cl.: A61K 31/27, A61K 31/167, A61P 31/12, A61P 31/18, A61K 31/196

(54) **Novel use of chemical compounds for the treatment of AIDS**

(71) Applicant: INSTITUT NATIONAL DE LA SANTE ET DE LA RECHERCHE MEDICALE (INSERM), 75013 Paris (FR); Centre National pour la Recherche Scientifique (CNRS), 75016 Paris (FR)
(72) Inventor: Collette, Yves, 13470 Carnoux-en-Provence (FR); Morelli, Xavier, 13009 Marseille (FR)
(74) Representative: Leszczynski, André

(57) **Abstract**

The invention relates to the use of compound of formula (I) as medicament: 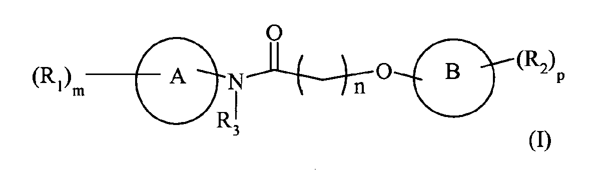 in the form of a free base or of an addition salt with an acid, as well as in the form of an hydrate or of a solvate.

The invention also relates to the use of such a compound of formula (I) for the preparation of a pharmaceutical composition intended for the treatment of AIDS.

The invention further relates to a medicament and to pharmaceutical composition comprising a compound of formula (I).

## Description

The instant invention relates to the use of some chemical compounds capable of binding to Human Immunodeficiency Virus Type I Nef protein (HIV-1 Nef).

The present invention particularly concerns the use of these compounds as inhibitors of interactions between Nef and its partners in infected cells, and thus as potential antiretroviral drug.

HIV-1 Nef has been shown to have a positive role in viral replication and pathogenesis (Fackler, O. T., Alcover, A. & Schwartz, O. (2007) Nat Rev Immunol 7, 310-7; Das, S. R. & Jameel, S. (2005) Indian J Med Res 121, 315-32; Peterlin, B. M. & Trono, D. (2003) Nat Rev Immunol 3, 97-107; Stevenson, M. (2003) Nat Med 9, 853-60). Being a non-enzymatic protein, Nef functions by means of protein-protein interactions, using distinct molecular surfaces such as the well-defined Src homology-3 (SH3) binding surface.

Nef is a viral protein that interacts with host cell signal transduction proteins to provide for long term survival of infected T cells and for destruction of non-infected T cells *(ibidem).*

Nef also advances the endocytosis and degradation of cell surface proteins, including CD4 and MHC proteins. CD4 is an integral membrane protein that functions in T-cell activation, and is the receptor for the HIV virus. This action possibly impairs cytotoxic T cell function, thereby helping the virus to evade the host immune response (Schwartz, et al., 1996, Nature Medecine 2: 338-342). The multifunctional protein thus helps the virus maintain high viral loads and overcome host immune defences, contributing to the progression of AIDS (Acquired Immune Deficiency Syndrom). Not surprisingly, persons infected with HIV-1 strains that have deletions of the Nef gene develop AIDS symptoms much more slowly than those infected with standard HIV (strains Fackler, O. T., Alcover, A. & Schwartz, O. (2007) Nat Rev Immunol 7, 310-7; Das, S. R. & Jameel, S. (2005) Indian J Med Res 121, 315-32; Peterlin, B. M. & Trono, D. (2003) Nat Rev Immunol 3, 97-107; Stevenson, M. (2003) Nat Med 9, 853-60). Nef may therefore be a valuable target for pharmaceutical intervention in AIDS progression (Saksela, K. (2004) Curr Drug Targets Immune Endocr Metabol Disord 4, 315-9).

Consequently, HIV-1 Nef protein constitutes an important therapeutic target in order to treat AIDS.

More particularly, the inventors of the instant invention have been interested in the interaction of Nef with macrophage-specific Src family member Hck. Actually, interaction between HIV-1 Nef and Hck (HIV-1 Nef/Hck) may be essential for M-tropic HIV replication and AIDS pathogenesis, identifying the virus host protein complex as a rational target for anti-HIV drug discovery (J. Mol. Biol. 2004, n°5, 343: 1255-1268).

Consequently, the purpose of the invention is to identify some chemical compounds which are capable of binding to HIV-1 Nef in order to interfere with its biological functions in viral infectivity, namely through the inhibition of its interactions with proteinic partners, such as SH3-containing Nef-binding partners and in particular with Hck, *via* binding to SH3 domain of Hck ((SH₃)Hck).

Inhibitors of protein-protein interactions are not so easy to identify as enzyme inhibitors, since most of the time, there lacks of a reference inhibitory compound (Pagliaro, L., Felding, J., Audouze, K., Nielsen, S.J., Terry, R.B., Krog-Jensen, C. and Butcher S. Cur. Opinion Chem. Biol. 2004, vol 8 p 442; Arkin, M. Cur. Opinion Chem. Biol 2005, vol 9, p 317).

WO 00/92778 describes cyclic chemical compounds, namely thiazolecarboxamide derivatives, as inhibitors of protein tyrosine kinases such as Lck, Fyn, Lyn, Hck, Fgr, Src, Yes, Blk, HER1 and HER2. Such inhibitors are useful for the treatment of protein tyrosine kinase-associated disorders such as immunologic and oncologic disorders.

WO 03/013523 discloses 2-aminopyridine, 2-aminoquinoline, 1-aminoisoquinoline derivatives, as potential ligand/inhibitor of SH3 domain of proteins, such as Hck.

More recently, Olszewski et al., (Proc. Natl. Acad. Sci. USA, 2004, 101(39): 14079-14084) describes the guanidine derivatives as useful protein-protein interaction inhibitor, namely between Nef and proteins such as p53, actine and Src kinase Lck. However, such compounds exhibit a high toxicity.

A different strategy avoiding the need of a first reference compound consists in selecting peptidic compounds as protein-protein interactions inhibitors. Such an approach has been disclosed in WO 2007/066018. However, peptidic compounds are not always easy to formulate and are often poorly "druggable".

Consequently, there is still needed to identify chemical compounds capable of selectively binding and inhibiting HIV-1 Nef/(SH3)Hck interaction, being at the same time biologically suitable for development as a medicine (decreased toxicity, high solubility).

Such an identification method has led the inventors to select chemical compounds, corresponding to the compound of formula (I), which are selective and turn out to be biologically suitable for development as a medicine (decreased toxicity, high solubility for example), namely for AIDS disease.

Consequently, a first object of the invention is the use of a compound of formula (I) as medicament: 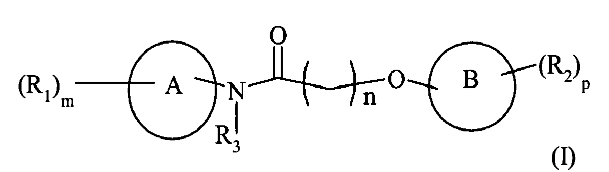 wherein:
- A and B represent, independently of each other, a mono- or bi-cyclic aryl group or a mono- or bi-cyclic heteroaryl group;
- m and p represent, independently of each other, a number having the value 0, 1, 2 or 3, being understood that when m represents 0, then no group R₁ is present on ring A and when p represents 0, then no group R₂ is present on ring B;
- R₁ represents:
   - an halogen atom,
   - a C₁₋₆ alcoxy,
   - -C(O)Rₐ wherein Rₐ represents an hydrogen atom, C₁₋₆ alkyl, said C₁₋₆ alkyl being optionally substituted with one or more halogen atoms or with one or more hydroxyl groups,
   - -C(O)OR_{b} wherein R_{b} represents an hydrogen atom, C₁₋₆ alkenyl, C₁₋₆ alkyl, said C₁₋₆ alkyl being optionally substituted with one or more halogen atoms or with one or more hydroxyl groups or else -C(O)OR_{b} is in its anionic form -C(O)O⁻,
   - hydroxyl,
   - -NR₄R₅, wherein R₄ and R₅ represent independently of each other an hydrogen atom, a C₁₋₃ alkyl,
   - -C(O)NR₄R₅, wherein R₄ and R₅ represent independently of each other an hydrogen atom, a C₁₋₃ alkyl,
   - tetrazolyl,
   - benzyloxy,
   - C₁₋₈ alkyl, said C₁₋₈ alkyl being optionally substituted with one ore more halogen atoms or with one or more hydroxyl groups, being understood that when m represents 2 or 3, then ring A is substituted with 2 or 3 groups R₁, wherein 2 or 3 groups R₁ represent independently of each other a group R₁ such as defined above;
- R₂ represents:
   - an halogen atom,
   - a C₁₋₆ alcoxy,
   - -C(O)Rₐ wherein Rₐ represents an hydrogen atom, C₁₋₆ alkyl, said C₁₋₆ alkyl being optionally substituted with one or more halogen atoms or with one or more hydroxyl groups,
   - -C(O)O R_{b} wherein R_{b} represents an hydrogen atom, C₁₋₆ alkenyl, C₁₋₆ alkyl, said C₁₋₆ alkyl being optionally substituted with one or more halogen atoms or with one or more hydroxyl groups or else -C(O)OR_{b} is in its anionic form -C(O)O⁻,
   - -NC(O)R_{c} wherein R_{c} represents an hydrogen atom, a C₁₋₆ alkyl,
   - hydroxyl,
   - C₁₋₈ alkyl, said C₁₋₈ alkyl being optionally substituted with one ore more halogen atoms or with one or more hydroxyl groups,
   - C₃₋₇ cycloalkyl,
   - mono- or bi-cyclic aryl,
   - mono- or bi-cyclic heteroaryl, being understood that when p represents 2 or 3, then ring B is substituted with 2 or 3 groups R₂, wherein 2 or 3 groups R₂ represent independently of each other a group R₂ such as defined above;
- n represents 0, 1, 2 or 3;
- R₃ represents a hydrogen atom, C₁₋₆ alkyl, C₃₋₆ cycloalkyl, an aryl group.

The compounds of formula (I) can comprise one or more asymmetric carbon atoms. They can therefore exist in the form of enantiomers or diastereoisomers. These enantiomers and diastereoisomers, as well as their mixtures, including racemic mixtures, form part of the invention.

The compounds of formula (I) can be provided in the form of a free base or in the form of their pharmaceutically acceptable salts. As used herein, the term "pharmaceutically acceptable salts" includes the acid addition salts of pharmaceutically acceptable mineral or organic acids as well as the salts of the compounds of formula (I). Among the salts of the compounds of general formula (I), there may be cited hydrochloric acid, hydrobromic acid, acetic acid, formic acid, propionic acid, oxalic acid, fumaric acid, maleic acid, succinic acid, benzoic acid, cinnamic acid, mandelic acid, citric acid, malic acid, tartaric acid, aspartic acid, glutamic acid, methanesulfonic acid, benzenesulfonic acid, p-toluenesulfonic acid, and the like. Such compounds of formula 51) in their form of their pharmaceutically acceptable salts also forms part of the invention.

The compounds of formula (I) can also exist in the form of an hydrate or of a solvate, i.e. in the form of associations or combinations with one or more water or solvent molecules. Such hydrates and solvates also form part of the invention.

The compound of formula (I) may also be in their cristallin, amorphous or oily forms, these forms being part of the invention. Such forms are also part of the invention.

The compounds of formula (I), which comprise an amino group can also exist in their N-oxydes form, and also form part of the invention.

The compounds of formula (I) of the instant invention also comprise the compounds wherein one or more hydrogen, carbon or halogen (namely chlorine or fluorine) atoms, have been replaced by their corresponding radioactive isotopes, such as tritium for replacing hydrogen atom or carbon 14 for replacing carbon 12. Such radiolabeled compounds are useful in research, namely in metabolism or pharmacocinetic studies, and in biological and pharmaceutical assays as biological/pharmaceutical tools.

According to the present invention, the terms below have the following meanings:
- a halogen atom corresponds to a fluorine, chlorine, bromine or iodine atom;
- in C₁₋₆, numeric subscripts indicate the possible number of carbon atoms (from 1 to 6 for instance), which are present in the group on which they relate. This meaning is also valuable for other values of numeric subscripts, C₁₋₆ being only an example.
- an alkyl group corresponds to a saturated, linear or branched aliphatic hydrocarbon group. For example, C₁₋₆ alkyl represents an hydrocarbon chain having from 1 à 6 carbon atoms, such as methyl, ethyl, propyl, isopropyl, butyl, isobutyl, tertbutyl, pentyl;
- an alkenyl group corresponds to an alkyl group such as defined above comprising at least one unsaturated bound;
- a cycloalkyl group corresponds to a saturated cyclic hydrocarbon group, optionally branched. The following examples may be cited: cyclopropyl, methylcyclopropyl, cyclobutyl, cyclopentyl, cyclohexyl. Such a cycloalkyl group may also be bridged, i.e. represents a group, which contains at least two rings that share a pair of bridgehead carbon atoms, for example bicycle[2.2.1]heptane;
- when an alkyl or a cycloalkyl group is substituted with one or more groups, these substituents may be present on the same carbon atom and/or on one or more carbon atoms;
- an alkoxy group corresponds to an -O-alkyl wherein an alkyl group is as defined above;
- an heterocyclic group: a saturated ring, comprising from 3 to 8 atoms and comprising at least one heteroatom such as nitrogen, sulfur or oxygen, or several heteroatom which are identical or different. Such a heterocyclic group may be morpholin, piperazin, pyrrolidin, piperidin, or azepin;
- an aryl corresponds to an aromatic, mono- or bi-cyclic ring comprising between 5 and 14 carbon atoms, preferably between 5 and 10. For example, an aromatic mono-cyclic group can be phenyl. For example, an aromatic bi-cyclic group can be azulen, naphtalen, inden;
- an heteroaryl group corresponds to an aromatic, mono- or bi-cyclic ring comprising between 5 and 14 carbon atoms, preferably between 5 and 10 and comprising between one or more heteroatoms, such as nitrogen, oxygen or sulfur atoms. When the heteroaryl is bi-cyclic, at least one of the ring is aromatic. Nitrogen atoms may be in the form of corresponding N-oxydes. A monocyclic heteroaryl can be thiazol, thiadiazol, thiophen, imidazol, triazol, tetrazol, pyridin, furan, oxazol, isoxazol, oxadiazol, pyrrol, pyrazol, pyrimidin, pyridazin, for example. A bi-cyclic heteroaryl can be indol, benzofuran, benzimidazol, benzothiophen, benzotriazol, benzothiazol, benzoxazol, quinolin, isoquinolin, indazol, quinazolin, phthalazin, quinoxalin, naphtyridin, 2,3-dihydro-1H-indol, 2,3-dihydro-benzofuran, 2,3-dihydro-inden, tetrahydroquinolin, tetrahydroisoquinolin, tetrahydroisoquinazolin, for example.
- a benzyloxy group : a group C₆H₅-O-, C₆H₅-CH₂-O-. Further objects of the invention will appear hereunder.

More particularly, the compound of formula (I) is useful for the preparation of a pharmaceutical composition intended to treat AIDS.

Advantageously, another object of the instant invention is the use of a subgroup of the compound of formula (I), which is represented by the compound of formula (Ia): 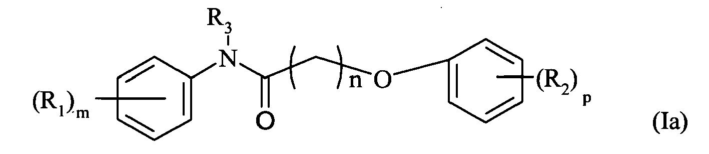 wherein R₁, R₂, R₃, m , n and p are as defined for the compound of formula (I).

Among the compounds of formula (I) or formula (Ia), another group of compounds which may be used according to the instant invention is defined such that m and p are at least equal to 1 and R₁ and R₂ are different from each other.

Advantageously, another group of compounds of formula (I) or (Ia) which may be used according to the instant invention is defined such that R₁ represents -C(O)OR_{b}, particularly -C(O)OR_{b} wherein R_{b} is an hydrogen atom or wherein -C(O)OR_{b} is in its anionic form -C(O)O⁻.

In a preferred manner, another subject-matter of the instant invention is represented by the compound of formula (I) or (Ia), wherein R₁ is in meta position and R₂ is in para position.

In a preferred manner, another object of the instant invention is represented by the compound of formula (I) or (Ia), wherein when m is 2, then at least one R₁ represents - C(O)OR_{b}.

Advantageously, another object of the instant invention is represented by the compound of formula (I) or (Ia), wherein R₂ represents a C₃₋₅ alkyl and more particularly a *ter*butyl.

Among the compounds of formula (I) or (Ia), another group of compounds which may be used according to the instant invention is defined such that:
- A and B represent an aryl group;
- R₁ represents an halogen atom, a -C(O)OR_{b} group wherein R_{b} is an hydrogen atom, an hydroxyl group or else R₁ represents a -C(O)O⁻,
- R₂ represents C₁₋₄ alkyl group;
- m = p = 1 or 2;
- R₃ and n are as defined for compound of formula (I).

Advantageously, in the previous group, R₁ represents -C(O)OR_{b} wherein R_{b} is an hydrogen atom; m is 1 or 2; R₂ represents a C₁₋₄ alkyl group; n represents 0 or 1 and R₃ represents an hydrogen atom.

Combinations of the preceding groups also form part of the invention.

According to the instant invention, some compounds of formula (I) are preferably used, which are:
- 2-hydroxy-5-[(4-*tert*-butylphenoxy)carbonylamino]benzoic acid of formula: 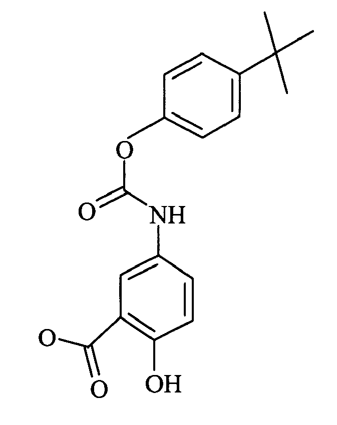
- 3-{[(4-tert-butylphenoxy)acetyl]amino}benzoic acid of formula:

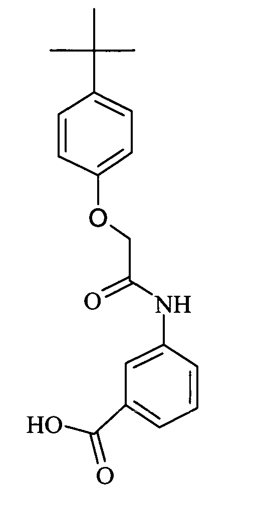

The compounds of formula (I) according to the instant invention are known and commercially available at Chembridge (www.chembridge.com) or can be obtained from the National Cancer Institute (NCI, Pubchem database for public research), or else may be prepared according to methods well-known by the person skilled in the art. For example: - 2-hydroxy-5-[(4-tert-butylphenoxy)carbonylamino]benzoic acid comes from Pubchem database (Identification number # 308963), and - 3-{[(4-*tert-*butylphenoxy)acetyl]amino}benzoic acid is available at NCI and comes from Chembridge Database (Identification number # 5744318).

Alternatively, the compounds of general formula (I) may be prepared by the person skilled in the art according to well-known methods in the art. For example, 2-hydroxy-5-[(4-tert-butylphenoxy)carbonylamino]benzoic acid and {[(4-*tert-*butylphenoxy)acetyl]amino}benzoic acid may be prepared by analogy with the methods using accessible scaffolds, such as described in Lee et al., J. Med. Chem (2007) 50, 1675-84).

In particular, the compound of formula (I), wherein n=0 may also be prepared by analogy with the method of preparation described in J. Med. Chem. 1987, vol. 30, pages 62 to 67.

The compounds of the invention can be used in therapeutic area and namely for the preparation of drugs, specifically of medicaments inhibiting protein-protein interaction, in particular HIV-1 Nef/(SH3)Hck interaction. Such compounds are thus of interest for the treatment of AIDS disease.

Therefore, one of the other aspects of the invention is a medicament, which comprises a compound of formula (I) or an addition salt thereof with a pharmaceutically acceptable salt, or a hydrate or solvate of a compound of formula (I).

As previously mentioned, these medicaments are useful in therapeutics, in particular in the treatment of AIDS and as an adjuvant to other treatment of AIDS, for example in combination with nucleoside analogue reverse transcriptase inhibitors (NARTIs or NRTIs), protease inhibitors and non-nucleoside reverse transcriptase inhibitors (NNRTI).

Another object of the invention is also a pharmaceutical composition, which comprises, as active principle, a compound according to the present invention. These pharmaceutical compositions comprise an effective dose of at least one compound according to the invention, or an addition salt thereof with a pharmaceutically acceptable salt, or an hydrate or solvate of the latter, and at least one pharmaceutically acceptable excipient.

Said excipients are chosen according to the pharmaceutical form and the administration route desired, among usual excipients known of one of skill in the art.

In the pharmaceutical compositions according to the invention for the oral, sublingual, sub-cutaneous, intramuscular, intra-venous, topical, local, intratracheal, intranasal, transdermal or rectal administration, the active principle of formula (I) above, its salt, solvate or hydrate, can be administered as a unitary dosage form, in blend with usual pharmaceutical excipients, to animals and human beings for the prevention or for the treatment of diseases mentioned above.

The appropriate unitary dosage forms comprise the oral forms, such as tablets, hard or soft gelatin capsules, powders, granules and oral solutions or suspensions, the sublingual, buccal, intratracheal, intraocular, intranasal forms, by inhalation, the topical, transdermal, sub-cutaneous, intramuscular or intra-venous forms, the rectal forms and the implants. For the topical application, the compounds of the invention may be used as creams, gels, ointments or lotions.

The amount of active principle to be administered will depend, as usual, on the nature and severity of the pathological conditions to be treated as well as on the weight of the patient and the administration route.

The present invention, according to another of its aspects, also relates to a method for the treatment of the above pathologies, which comprises the administration to a patient of an effective dose of a compound according to the invention, or a salt with a pharmaceutically acceptable salt thereof, or an hydrate or a solvate thereof.

These compounds have been tested *in vitro.* Experimental protocols and results are detailed in the following examples.

Such examples only illustrate the purpose of instant invention and do not restrict it. In the following examples/figures:
- DMSO : dimethyl sulfoxid
- MFI : Mean Fluorescence Intensity
- GFP : Green Fluoroscent Protein
- GFP^{high}: ibidem (high fluorescence)
- EDTA: ethylene glycol tetracetic acid
- EGTA: ethylene glycol-bis(beta-aminoethyl ether)-N,N,N',N'-tetracetic acid OK
- GST: glutathione sulfo transferase
- PVDF membrane:Polyvinylidene fluoride
- 2-hydroxy-5-[(4-tert-butylphenoxy)carbonylamino]benzoic acid referred to Compound X,
- 3-{[(4-tert-butylphenoxy)acetyl]amino}benzoic acid referred to Compound Y.

### Description of the drawings referred to in Examples 1, 2 and 3.

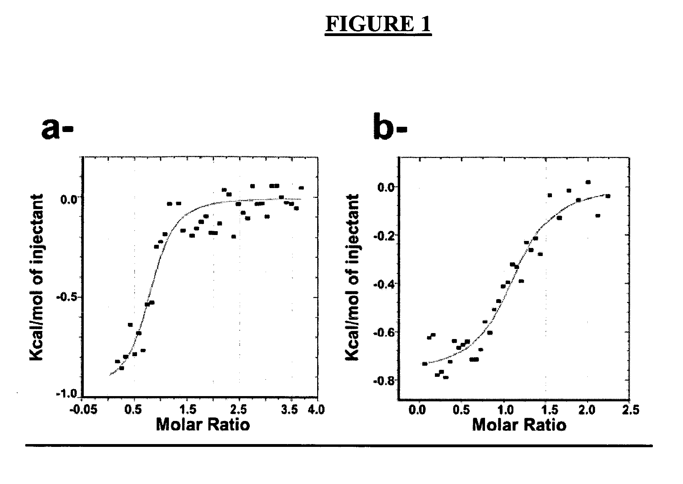

- Figure 1 represents Isothermal Titration Calorimetry (ITC) results.

Experimental binding curves (black squares) and fit are displayed for the interaction between Nef57-205 and *a)* Compound X and b) Compound Y. Error bars represent the mean +/- SD for three independent experiments with cells isolated from at least three independent blood donors.

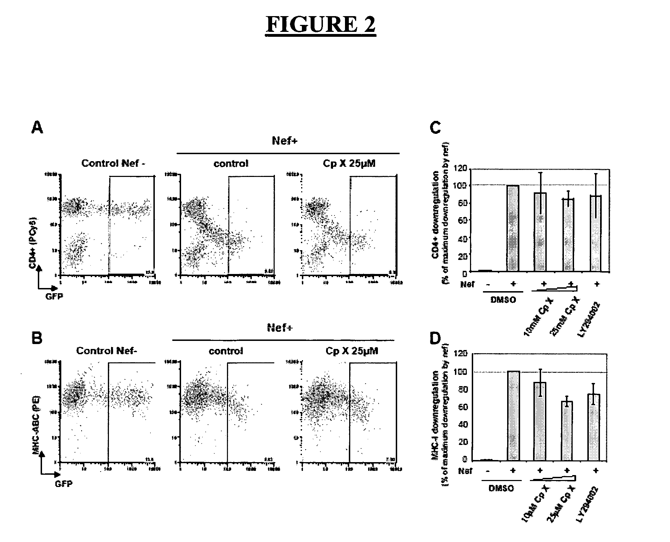

- Figure 2 represents MHC-I down-regulation in human peripheral blood mononuclear cells (PBMC) by Nef in presence of Compound X. Right side panels A and B represent quiescent PBMC transfected with Nef-GFP expression plasmid, treated with or without (control, i.e. 0,5 % DM50) 25µM of Compound X, and left side panels A and B represent PBMC transfected with GFP expression plasmid (control Nef). Panel A shows the results when cells were then incubated with human anti-CD4⁺ - PE and panel B shows the results when cells were then incubated with human anti-HLA-ABC (MCH-I). Viable CD4+T cells were analysed for GFP and anti-CD4 (panel A) or GFP and anti-HLA-ABC (panel B) staining. The frequency of GFP^{high} cells displaying down-regulated CD4 + or MHC I is shown in the right parts (right "boxes") of each of the three panels A and B. The Nef⁺/Nef MFIs ratio calculated in these "boxes" in untreated control cells was normalized as 100%. Nef mediated CD4⁺ or MHC-I down regulation is presented in panels C and D, respectively.

Error bars represent the mean +/- SD for three independent experiments with cells isolated from at least three independent blood donors.

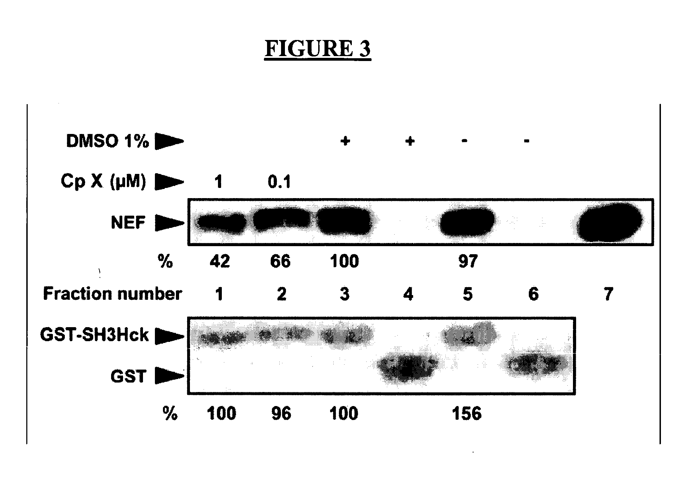

- Figure 3: GST-pulldown assay Nef57-205 was incubated with Compound X or vehicle (DMSO), and then reacted with GST (lanes 4, 6) or GST-SH3Hck (lanes 1-3, 5) followed by SDS-PAGE resolution of protein complexes and transferred to a PVDF membrane. Nef protein was detected by immunoblotting and chemiluminescence (upper panel). Equivalent GST loading was verified by Red Ponceau staining (lower panel). Results were quantified and normalized to fraction number 3 fixed as 100% signal.

Nef protein was also directly loaded on the gel as loading control (lane 7).

### Sequence variability analysis on 1292 Nef sequences

1292 Nef protein sequences from the Los Alamos HIV sequence Database has been considered. This alignment has been treated in order to build a Nef frequency amino acid position matrix for the entire population sample using a Microsoft Excel computing table. This frequency matrix was then used to build the best conserved Nef sequence with the most frequent residues at each position. This sequence was then compare to the Nef protein sequence (B.FR.83.HXB2_LAI_IIIB_BRU_K03455 from the Los Alamos Natl lab, www.hiv.lanl.gov) used for the *in vitro*/cellular assays to ensure that work was performed on a representative model of the targeted surface

### Example 1: Isothermal Titration Calorimetry (ITC) Experiments with Compounds X and Y (figure 1)

Isothermal titration calorimetry (ITC) is used to investigate *in vitro* the binding affinity and thermodynamic parameters of the inhibitory molecules.

Purified Nef57-205, as described in *Arold et al, Structure* 1997 5(10) p 1361) was extensively dialyzed in degassed ITC buffer (20 mM Hepes pH 7.0, 100 mM NaCl, 5% DMSO), except for binding experiments with Compound Y, where 20 mM Na-phosphate pH 8.0, 100 mM NaCl, 5% DMSO was used as buffer. Synthetic inhibitors were first dissolved at 10 mM in 100% DMSO, and then diluted to 300 - 400 µM in ITC buffer with DMSO concentration adjusted to 5%. Inhibitors were injected from the 300 µL syringe into the 1.4 mL sample cell containing 15-25 µM Nef57-205. ITC titrations were performed at 25 °C, using 10 - 15 µl injections every 300 secondes. All inhibitors were also titrated into ITC buffer alone, and the resulting heat of dilution was subtracted from the experimental curves. ITC was performed with a VP ITC MicroCalorimeter from MicroCal Inc., and data were fitted with Microcal Origin^{™} software. The results are summarized in Table I and illustrated in Figure 1.

**Table I**

| **Compound** | **N** | **k_{D}** (µM) | Δ**G** (kJ/mol) | Δ**H**(kJ/mol) | **T**Δ**S**(kJ/mol) |
|---|---|---|---|---|---|
| **X** | 0.83 ± 0.06 | 1.8 ± 0.85 | -32.8 | -4.0 ± 0.4 | 28.8 |
| **Y** | 1.14 ± 0.1 | 0.98 ± 0.3 | -34.2 | -3.2 ± 0.20 | 31.0 |

In Table I:
- errors were taken from least square fitting, and do not take into account experimental uncertainties of small compound concentrations.
- * Indicates that the stoichiometry (N) needed to be fixed to unity to allow data analysis.
- ΔG represents: free enthalpy variation
- ΔH represents: enthalpy variation
- TΔS represents: entropy variation

The results in Table I show that upon binding to Nef57-205, compounds of the instant invention displayed very similar characteristics, namely a stoichiometry close to one, and a micro-molar K_{D}, resulting from a small favourable enthalpy, and large favourable entropy.

A favourable entropy in binding can result from a gain of motional freedom, and/or hydrophobic interactions (reviewed in Ladbury & Chowdhry, Chem Biol 3, 973-80 (1996)). The small but significant enthalpic contribution suggests formation of hydrogen bonds and/or van der Waals or ionic interactions.

Enthalpy and entropy data show the existence of energy bounds between Nef and the compounds of formula (I) of the invention, thus confirming interactions between Nef and compounds of the invention.

The stoichiometry data show an equimolar interaction between Nef and the compounds of the invention, since N is close to 1.

As shown in Table I, the apparent K_{D} of the compound according to the invention is in the micromolar range. For example:
- Compound X has a K_{D} of 1.8 ± 0.85 µM, and
- Compound Y has a K_{D} of 0.98 ± 0.3 µM.

The compounds of the invention exhibit a K_{D}, which is comparable to the affinity of the Nef. SH₃Hck interaction (K_{D} = 0.25 µM as disclosed in Lee and al. (1995), EMBO J., 14, 5006-15).

More particularly, the compound according to the instant invention exhibit a K_{D} which is comprised between 10 nM and 10 µm, preferably between 10 nM and 5 µM, more preferably between 100 nM and 2 µM.

The results of Table I, i.e. equimolar stoichiometry (1:1), favourable energy bounds and low K_{D}, clearly show that the compounds according to the invention interact and efficiently bind to Nef protein.

### Example 2: MHC-I down-regulation in human peripheral blood mononuclear (PBMC) cells by Nef in presence of Compound X (figure 2)

Nef advances the endocytosis and degradation of all surface proteins, including CD4 and MHC proteins (Schwartz et al., 1996, Nature Medecine 2:338-342). Consequently, some compounds of the instant invention were tested for their ability to prevent their degradations by interacting with Nef. Human peripheral blood mononuclear cells (PBMC) from healthy volunteers were purified by Ficoll gradient centrifugation ( Lymphoprep^{™}, Axis-shield). Washed, isolated PBMC were transiently transfected using the nucleofection technology (human T cell Nucleofector^{™} kit, 5.10⁶ cells, 5µg plasmid DNA Nef-GFP or GFP (Nucleofactor Program V-024^{™}, Amaxa Biosystems)). The same Nef sequence as described above in example 1 was cloned in pEGFP-N3 (Clonetech). Immediately after nucleotransfection, cells were transferred to 12 well plates containing pre-warmed culture medium (500µl RMPI 1640 supplemented with 10% fetal bovine serum (FBS)). Two days following nucleofection, cells were treated 3 hr with Compound X (25µM or 10µM) or 5µM LY294002 (Calbiochem). LY 29002 is used here as control for inhibition of Nef-mediated MHCI down-regulation as described in Hung et al 2007.

At 45 hr post transfection cells were treated with 25 µM of Compound X or 0.5% DMSO (control) for 3 hrs. Cells were then incubated with human anti-CD4+-PE or human anti-HLA-ABC (MHC-I) and analyzed by flow cytometry. Flow Cytometric Analysis were performed as follows: Cells were washed and resuspended in fluorescence-activated cell sorter (FACS) medium (PBS 2% FCS 0.02% NaN3, pH 7.2). Cells were incubated with phycoerythrin (PE) anti-human HLA-ABC (clone G46-2.6, BD Pharmingen, (ref. 555-553)) or PE Cyanine 5 anti-human CD4 (clone RPA T4, BD Pharmingen (ref. 555-348)) at 4°C for 1 hr. An isotype-matched antibody was used as a negative control (Beckman Coulter for 20100326) for PE-HLA-ABC and BD Pharmingen (ref. 551497) was used as control for PE Cy5 CD4. Cells were washed and analyzed on FACS scan using Flow Jo software^{™} (Beckman Coulter for 20100326 and BD Pharmingen for 551497).

Viable CD4+ T cells were analyzed for GFP and anti-CD4+ (panel A, figure 2) or GFP and anti-HLA-ABC (panel B, figure 2) staining. The frequency of GFP^{high} cells displaying down-regulated CD4+ or MHC-I is shown in the right gate (panels A and B, figure 2). The Nef+/Nef- MFIs ratio measured in this gate in untreated control cells was normalized as 100% Nef-mediated CD4+ or MHC-I down-regulation (panel C and D, respectively in the figure 2). The Nef+/Nef- MFIs ratio calculated in treated cells was plotted relative to these control cells value and thus indicated the extend of Nef-mediated CD4 and MHC-I down-regulation when treated with a compound according to the instant invention.

Cell treatment with Compound X also reduce in a dose-dependent manner Nef-mediated MCH-1 but not CD4 down-regulation, which are respectively dependent and independent on the Nef-SH3 binding surface, as efficiently as the PI3K inhibitor LY294002 (Hung et al., (2007) Cell. Host & Microbe 1, 121-133).

Accordingly, the results show that MHC-I down-regulation in human PBMC cells by Nef is partially blocked in presence of Compound X.

This test shows that compounds according to the invention efficiently inhibit Nef interactions with proteic partners.

### Example 3: GST pull-down assay (figure 3)

100 ng of purified Nef57-205 (the same Nef sequence as described above in example 1 was cloned in pEGFP-N3 (Clonetech), according to the manufacturer's instructions) was pre-incubated at 4°C with inhibitor sample (Compound X) or vehicle (DMSO 1%) in 500 µL of reaction buffer (25 mM Hepes, pH 7.8, 150 mM NaCl, 10 mM EDTA, 1 mM EGTA, 1% Triton X-100), followed by addition of Sepharose-coupled recombinant GST or GST-SH3Hck (29 µg/10 µL) for 2 h at 4°C. After 3 washes using reaction buffer, protein complexes were resolved by SDS-PAGE, transferred to a PVDF membrane, coloured by Red Ponceau to visualize loaded GST proteins, and the amount of Nef protein bound to GST matrix was detected by anti-Nef western blotting and chemiluminescence (SuperWest Pico, PIERCE, Perbio, France) as described in Greenway, et al. (1999) J Virol 73, 6152-8). Bands intensity was quantified using ImageJ (NCI).

This test shows that the competition between two proteic partners is dose-depending.

The results obtained from examples 1, 2 and 3 demonstrate that the compounds according to the instant invention selectively bind Nef SH3 domain (in the µM range) and can functionally compete for SH3Hck interaction, *both in vitro* and in cell-based assays and thus display inhibition activity towards protein-protein interaction, in particular HIV-1 Nef/(SH3)Hck interaction.

In view of the tests results, the compounds of the invention constitute interesting therapeutic agents in order to combat HIV-1 virus and thus to treat or co-treat AIDS.

## Claims

1. Use of compound of formula (I) as medicament: 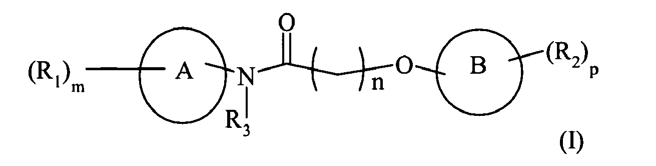 wherein:
- A and B represent, independently of each other a mono- or bi-cyclic aryl group or a mono- or bi-cyclic heteroaryl group;
- m and p represent independently of each other a number having the value 0, 1, 2 or 3, being understood that when m represents 0, then no group R₁ is present on ring A and when p represents 0, then no group R₂ is present on ring B;
- R₁ represents:
• an halogen atom,
• a C₁₋₆ alcoxy,
• -C(O)Rₐ wherein Rₐ represents an hydrogen atom, C₁₋₆ alkyl, said C₁₋₆ alkyl being optionally substituted with one or more halogen atoms or with one or more hydroxyl groups,
• -C(O)OR_{b} wherein R_{b} represents an hydrogen atom, C₁₋₆ alkenyl, C₁₋₆ alkyl, said C₁₋₆ alkyl being optionally substituted with one or more halogen atoms or with one or more hydroxyl groups or else -C(O)OR_{b} is in its anionic form -C(O)O⁻,
• hydroxyl,
• -NR₄R₅, wherein R₄ and R₅ represent independently of each other an hydrogen atom, a C₁₋₃ alkyl,
• -C(O)NR₄R₅, wherein R₄ and R₅ represent independently of each other an hydrogen atom, a C₁₋₃ alkyl,
• tetrazolyl,
• benzyloxy,
• C₁₋₈ alkyl, said C₁₋₈ alkyl being optionally substituted with one ore more halogen atoms or with one or more hydroxyl groups, being understood that when m represents 2 or 3, then ring A is substituted with 2 or 3 groups R₁, wherein 2 or 3 groups R₁ represent independently of each other a group R₁ such as defined above;
- R₂ represents:
• an halogen atom,
• a C₁₋₆ alcoxy,
• -C(O)Rₐ wherein Rₐ represents an hydrogen atom, C₁₋₆ alkyl, said C₁₋₆ alkyl being optionally substituted with one or more halogen atoms or with one or more hydroxyl groups,
• -C(O)OR_{b} wherein R_{b} represents an hydrogen atom, C₁₋₆ alkenyl, C₁₋₆ alkyl, said C₁₋₆ alkyl being optionally substituted with one or more halogen atoms or with one or more hydroxyl groups or else -C(O)OR_{b} is in its anionic form -C(O)O⁻,
• -NC(O)R_{c} wherein R_{c} represents an hydrogen atom, a C₁₋₆ alkyl,
• hydroxyl,
• C₁₋₈ alkyl, said C₁₋₈ alkyl being optionally substituted with one ore more halogen atoms or with one or more hydroxyl groups,
• C₃₋₇ cycloalkyl,
• mono- or bi-cyclic aryl,
• mono- or bi-cyclic heteroaryl, being understood that when p represents 2 or 3, then ring B is substituted with 2 or 3 groups R₂, wherein 2 or 3 groups R₂ represent independently of each other a group R₂ such as defined above;
- n represents 0, 1, 2 or 3;
- R₃ represents a hydrogen atom, C₁₋₆ alkyl, C₃₋₆ cycloalkyl, an aryl group, in the form of a free base or of an addition salt with an acid, as well as in the form of an hydrate or of a solvate.

2. Use of a compound of formula (Ia), according to the preceding claim, wherein compound of formula (Ia) is: 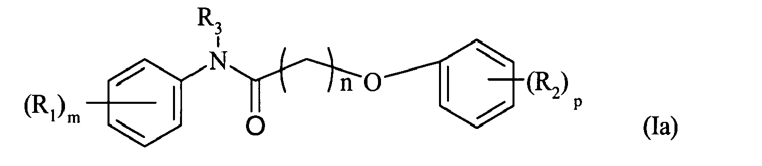 wherein R₁, R₂, R₃, m, n and p are as defined for the compound of formula (I).

3. Use according to any of the preceding claims, wherein m and p are at least equal to 1 and R₁ and R₂ are different from each other.

4. Use according to any of the preceding claims, wherein R₁ represents - C(O)OR_{b}.

5. Use according to the preceding claim, wherein R_{b} is an hydrogen atom or wherein -C(O)OR_{b} is in its anionic form -C(O)O⁻.

6. Use according to any of the preceding claims, wherein R₁ is in meta position and R₂ is in para position.

7. Use according to any of the preceding claims, wherein, when m is 2, then at least one R₁ represents -C(O)OR_{b}.

8. Use according to any of the preceding claims, wherein R₂ represents a C₃₋₅ alkyl.

9. Use according to any of the preceding claims, wherein:
- A and B represent an aryl group;
- R₁ represents an halogen atom, a -C(O)OR_{b} group wherein R_{b} is an hydrogen atom, an hydroxyl group or else R₁ represents a -C(O)O⁻,
- R₂ represents C₁₋₄ alkyl group;
- m = p = 1 or 2;
- R₃ and n are as defined for compound of formula (I).

10. Use according to any of the preceding claims of a compound chosen among:
• 2-hydroxy-5-[(4-tert-butylphenoxy)carbonylamino]benzoic acid of formula: 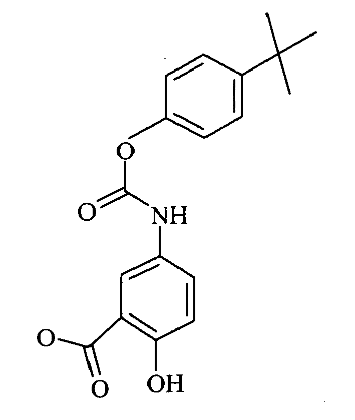
• 3-{[(4-*tert-*butylphenoxy)acetyl]amino}benzoic acid of formula:

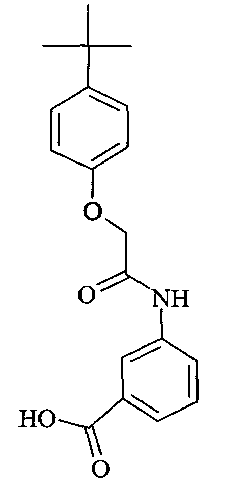

11. Use of a compound as defined in claims 1 to 10, for the preparation of a pharmaceutical composition intended for the treatment of AIDS.

12. Medicament, comprising a compound as defined in claims 1 to 10, or an addition salt of said compound to a pharmaceutically acceptable salt, or an hydrate or solvate of said compound.

13. Pharmaceutical composition, comprising a compound as defined in claims 1 to 10, or an addition salt of said compound to a pharmaceutically acceptable salt, or an hydrate or solvate of said compound, and at least one pharmaceutically acceptable excipient.
